# EUROPEAN PATENT SPECIFICATION

(11) **EP 1 206 411 B1**
(45) Date of publication and mention of the grant of the patent: **17.10.2007**
(21) Application number: 00953580.8
(22) Date of filing: 15.08.2000
(51) Int. Cl.: C01C 1/24, C07D 201/16

(54) **PROCESS FOR TREATING A MIXTURE COMPRISING AN AMMONIUM SULFATE SOLUTION PHASE AND AN AQUEOUS LACTAM PHASE**
VERFAHREN ZUR BEHANDLUNG EINER AMMONIUMSULFATLÖSUNG UND EINE WÄSSRIGE LACTAMPHASE ENTHALTENDES GEMISCH
METHODE DE TRAITEMENT D'UN MELANGE COMPRENANT UNE PHASE EN SOLUTION DE SULFATE D'AMMONIUM ET UNE PHASE LACTAME AQUEUSE

(30) Priority: 17.08.1999 EP 99202667
(43) Date of publication of application: 22.05.2002
(73) Proprietor: DSM IP Assets B.V., 6411 TE Heerlen (NL)
(72) Inventor: LEMMENS, Johannes, Albertus, Wilhelmus, NL-6041 HV Roermond (NL); LANSU, Anne, Maria, Arthur, NL-6129 GG Urmond (NL); EKKELENKAMP, Geert, NL-6121 JH Born (NL); DEBEIJ, Johannes, Elisabeth, Pierre, NL-6269 NS Margraten (NL)
(86) International application number: PCT/NL2000/000572
(87) International publication number: WO 2001/012549

(56) References cited:
- DD-A- 287 258
- GB-A- 1 353 448
- US-A- 3 937 789
- US-A- 5 637 700
- DATABASE WPI Section Ch, Week 199101 Derwent Publications Ltd., London, GB; Class C04, AN 1991-003035 XP002128416 & JP 02 279514 A (UBE IND LTD), 15 November 1990 (1990-11-15) cited in the application
- DATABASE WPI Week 197439 Derwent Publications Ltd., London, GB; AN 1974-6871 XP002128417 & JP 49 032716 B (SUMITOMO), 2 September 1974 (1974-09-02)

## Description

The invention relates to a process for treating a mixture, in particular a neutralized Beckmann rearrangement mixture, comprising i) an ammonium sulfate solution phase containing first organic impurities; and ii) an aqueous lactam phase containing second organic impurities.

Lactam may be prepared by subjecting a cyclic ketoxime to the Beckmann rearrangement in the presence of sulphuric acid or oleum, resulting in a Beckmann rearrangement mixture. The Beckmann rearrangement mixture may be neutralized with ammonia or ammonium base. The neutralized Beckmann rearrangement mixture typically comprises an ammonium sulfate solution phase and an aqueous lactam phase. The ammonium sulfate concentration in the ammonium sulfate solution phase is generally between 25 an 50 wt.%. The lactam concentration in the aqueous lactam phase is generally between 60 and 80 wt.%. Typically, organic impurities are present in both phases. In the processing of the neutralized Beckmann rearrangement mixture, the organic impurities are often discarded in one or more waste streams, which often also include ammonium sulfate. See for examples GB-A-1353448.

In CN-C-1023790 a process is described for treating an ammonium sulfate solution phase of a neutralized Beckmann rearrangement mixture. Ammonium sulfate crystals are recovered from the ammonium sulfate solution phase, and organic impurities originating from the ammonium sulfate solution phase are oxidized in a wet air oxidation process. Ammonium sulfate crystals are recovered from the ammonium sulfate solution phase using a crystallizer which is operated by evaporation. Mother liquor which contains organic impurities, is removed from the crystallizer and treated in a wet air oxidation process. In the wet air oxidation process the mother liquor is contacted with an oxygen-containing gas in a wet air oxidation reactor, designated as oxygenolysis column, resulting in gaseous oxidized products and purified ammonium sulfate solution. The purified ammonium sulfate solution, which has a decreased amount of organic impurities, is recycled to the crystallizer. In the process of CN-C-1023790 no treatment is provided for waste containing organic impurities originating from the aqueous lactam phase.

JP-B-49032716 discloses a process for treating a neutralized Beckmann rearrangement mixture. After separation of the lactam phase, the ammonium sulfate solution is heat-treated at from 150 to 350 °C under increased pressure in oxygen or gas which contains oxygen. JP-B-49032716 does not disclose a treatment for organic impurities in the aqueous lactam phase.

It is the aim of the invention to provide a process for treating a mixture, in particular a neutralized Beckmann rearrangement mixture, comprising i) an ammonium sulfate solution phase containing first organic impurities and ii) an aqueous lactam phase containing second organic impurities, in which process the amount of waste containing organic impurities is reduced in an efficient manner.

According to the invention the process comprises forming an aqueous liquid containing the first and second organic impurities, and subjecting the aqueous liquid to a wet air oxidation process to purify the aqueous liquid.

Liquid containing organic impurities originating from the ammonium sulfate solution phase and from the aqueous lactam phase and ammonium sulfate can be purified and recycled to a crystallizer. A significant reduction of the amount of waste which contains ammonium sulfate and organic impurities as well as an increased recovery of ammonium sulfate as crystals can be effected according to the invention. Ammonium sulfate crystals with good quality can be obtained.

There are many possibilities to recover the aqueous liquid from the mixture, in particular from the neutralized Beckmann rearrangement mixture.

In a preferred embodiment (embodiment A) the process comprises the step of:
Aa) contacting the mixture with an organic solvent, resulting in a lactam-containing organic liquid and an ammonium sulfate solution.
   As a result of step Aa) lactam is extracted, and an ammonium sulfate solution is obtained containing the first and second organic impurities. The ammonium sulfate solution obtained may be subjected to a wet air oxidation process. Purified solution may then be fed to a crystallizer, and mother liquor withdrawn from the crystallizer may be recycled to the wet air oxidation process. More preferably, the process comprises the steps of:
Ab) processing the ammonium sulfate solution through a crystallizer to form ammonium sulfate crystals and a mother liquor
Ac) combining the mother liquor with aqueous dilution liquid, resulting in an oxidation mixture
Ad) subjecting the oxidation mixture to a wet air oxidation process, resulting in purified ammonium sulfate solution.

This has the advantage that the organic impurities are concentrated before they are fed to the oxidation reactor, so that a smaller oxidation reactor may be used. Step Ac) effects that an oxidation mixture is obtained having a lower ammonium sulfate concentration than the mother liquor, this being advantageous since it minimizes and preferably prevents the occurrence of crystallization in the purified ammonium sulfate solution, while or after leaving the oxidation reactor.

Most preferably, the process comprises the step of:
Ae) recycling the purified ammonium sulfate solution to the crystallizer.

In another preferred embodiment (B) the process comprises the steps of:
Ba) separating the ammonium sulfate solution phase and the aqueous lactam phase, resulting in an ammonium sulfate solution and aqueous lactam
Bb) contacting the aqueous lactam with an organic solvent, resulting in a lactam-containing organic liquid and an aqueous effluent
Bc) combining the ammonium sulfate solution with the aqueous effluent.

As a result of step Bb) lactam is extracted, and an aqueous effluent is obtained containing organic impurities (second organic impurities). As a result of step Bc) a combined ammonium sulfate solution and aqueous effluent is obtained, containing the first and second organic impurities. Step Bc) may be carried out by feeding the ammonium sulfate solution and the aqueous effluent to the same crystallizer or the same wet air oxidation reactor. Preferably, small amounts of lactam which may have remained in the ammonium sulfate solution and/or the aqueous effluent are extracted from the ammonium sulfate solution and/or aqueous effluent using an organic solvent. The combined ammonium sulfate solution and aqueous effluent may be subjected to a wet air oxidation process. Purified solution may then be fed to a crystallizer, and mother liquor withdrawn from the crystallizer may be recycled to the wet air oxidation process. More preferebly, the process comprises the steps of:
Bd) processing the combined ammonium sulfate solution and aqueous effluent through a crystallizer to form ammonium sulfate crystals and a mother liquor
Be) combining the mother liquor and aqueous dilution liquid, resulting in an oxidation mixture
Bf) subjecting the oxidation mixture to a wet air oxidation process, resulting in purified ammonium sulfate solution.

This has the advantage that the organic impurities are concentrated, before they are fed to the oxidation reactor, so that a smaller oxidation reactor may be used. Step Be) effects that an oxidation mixture is obtained having a lower ammonium sulfate concentration than the mother liquor, this being advantageous since it minimizes and preferably prevents the occurrence of crystallization in the purified ammonium sulfate solution, while or after leaving the oxidation reactor.

Most preferably, the process comprises the step of:
Bg) recycling the purified ammonium sulfate solution to the crystallizer.

In another preferred embodiment (C) the process comprises the steps of:
Ca) separating the ammonium sulfate solution phase and the aqueous lactam phase, resulting in an ammonium sulfate solution and an aqueous lactam
Cb) contacting the aqueous lactam with an organic solvent, resulting in a lactam-containing organic liquid and an aqueous effluent
Cc) processing the ammonium sulfate solution through a crystallizer to obtain ammonium sulfate crystals and a mother liquor
Cd) combining the mother liquor and the aqueous effluent, resulting in an oxidation mixture
Ce) subjecting the oxidation mixture to a wet air oxidation process, resulting in purified ammonium sulfate solution.

As a result of step Cb) lactam is extracted, and an aqueous effluent is obtained containing organic impurities (second organic impurities). As a result of step Cd) an oxidation mixture is obtained containing the first and second impurities. Embodiment (C) has the advantage that the flow rate of the mother liquor removed from the crystallizer can be kept smaller, without raising the concentration of organic impurities in the crystallizer. A small flow rate of mother liquor removed from the crystallizer has the advantage that a small oxidation reactor can be used. Combining the mother liquor with aqueous effluent having a lower ammonium sulfate content than the mother liquor has also the advantage that the ammonium sulfate concentration of the oxidation mixture is decreased, so that no, or at least less aqueous dilution liquid is needed from external sources. Decreasing the amount of aqueous dilution liquid which does not originate from the effluent has also the advantage that less water is evaporated in the crystallizer and/or oxidation reactor, which saves energy. Combining the mother liquor with aqueous effluent, which contains organic impurities, has the advantage that the COD content of the oxidation mixture is higher than in the case that the mother liquor is diluted with aqueous dilution liquid not containing organic impurities. Preferably, the COD content of the oxidation mixture is increased with respect to the COD content of the mother liquer. An increased COD content of the oxidation mixture has the advantage that less energy is needed to operate the oxidation reactor. If the COD content of the oxidation mixture is sufficiently high, it is even possible that the oxidation reactor can be operated without the use of energy from external sources, or that a surplus of energy is produced. As used herein, the values for the COD (chemical oxygen demand) content, which is a measure for the concentration organic impurities, refers to values as determined according to ASTM D 1252-95 (dichromate method).

Most preferably, the process comprises the step of:
Cf) recycling the purified ammonium sulfate solution to the crystallizer.

In another preferred embodiment (D), the process comprises the steps of:
Da) separating the ammonium sulfate solution phase and the aqueous lactam phase, resulting in an ammonium sulfate solution and an aqueous lactam
Db) contacting the aqueous lactam with an organic solvent, resulting in a lactam-containing organic liquid and an aqueous effluent
Dc) processing the ammonium sulfate solution through a crystallizer to obtain ammonium sulfate crystals and mother liquor
Dd) combining the mother liquor with the mixture
De) subjecting the aqueous effluent to a wet air oxidation process, resulting in purified effluent.

As a result of step Dd), organic impurities present in the mother liquor and originating from the ammonium sulfate solution phase (first organic impurities) enter the aqueous lactam phase. As a consequence aqueous lactam and aqueous effluent are obtained containing the first and second impurities. Embodiment (D) has the advantage that a small reactor may be used, since only the effluent needs to be treated in the oxidation reactor.

More preferably, the process comprises the step of:
Df) recycling the purified effluent to the crystallizer.

The wet air oxidation process is preferably carried out by contacting the aqueous liquid containing the first and second impurities, also referred to as oxidation mixture (embodiments A, B, C) or aqueous effluent (embodiment D), with an oxygen-containing gas at elevated temperature and elevated temperature in an oxidation reactor. The aqueous liquid may include two or more combined liquids, such as for instance mother liquor and aqueous dilution liquid in the case of embodiments A and B or mother liquor and aqueous effluent in the case of embodiment C. Said liquids may be fed to the oxidation reactor as separate flows, in which case they are combined in the oxidation reactor. Preferably, they are combined, before they are fed to the oxidation reactor. It is known to the skilled person how a wet air oxidation process may be carried out, see for instance CN-C-1023790. Usually, the aqueous liquid is pressurized, after which it is combined with pressurized oxygen-containing gas. The amount of oxygen-containing gas used usually depends on the COD content of the feed of the oxidation reactor and can be determined by the skilled person. Usually, the combined oxidation mixture and oxygen-containing gas is subsequently heated in a heat exchanger, after which it is fed into the oxidation reactor. The oxidation reactor, which is preferably a bubble column, is usually operated at a pressure between 5 and 200 bar, preferably between 20 and 170 bar. The oxidation reactor is usually operated at a temperature between 125 and 320 °C, preferably between 175 and 320 °C, more preferably between 200 and 300 °C. Increasing the temperature generally results in an increased conversion of organic impurities into gaseous oxidized products. In general a higher pressure is used if the oxidation reactor is operated at a higher temperature. The oxygen containing gas may be pure molecular oxygen. However, the oxygen containing gas is preferably a mixture of oxygen with nitrogen, such as for instance air. The concentration of molecular oxygen in the oxygen containing gas is usually 5 to 50 vol.%, preferably 10 to 40 vol.%. In the oxidation reactor organic impurities are oxidized, resulting in gaseous oxidized products, typically CO₂ and a purified aqueous liquid, also referred to as purified ammonium sulfate solution or purified effluent. The oxidation reaction may be carried out with or without catalyst. Carrying out the oxidation without catalyst is preferred. This results in a better quality of the ammonium sulfate crystals, in case the purified ammonium sulfate solution is recycled to a crystallizer.

In a preferred embodiment, the average COD content of the feed of the oxidation reactor is at least 20 g/kg, more preferably at least 45 g/kg. The feed of the oxidation reactor is understood to be all liquids which are fed to the oxidation reactor, and which are to be contacted with the oxygen containing gas. An increased COD content of the feed of the oxidation reactor has the advantage that less energy from external sources is needed to operate the oxidation reactor. If the COD content of the feed of the oxidation reactor is sufficiently high, the oxidation reactor may even be operated without the use of energy from external sources. If the COD content of the feed of the oxidation reactor further increases a surplus of energy is produced. Preferably, the COD content of the feed of the oxidation reactor is lower than 300 g/kg.

As a result of the occurrence of evaporation of water during the oxidation process or during the separation of the purified ammonium sulfate solution and gaseous oxidized products, crystallization in the purified aqueous liquid may occur if the ammonium sulfate concentration in the feed of the oxidation reactor is not sufficiently low. The occurrence of crystallization in the purified aqueous liquid may give rise to clogging problems. Preferably, the ammonium sulfate concentration of the feed of the oxidation reactor is sufficiently low that crystallization in the purified aqueous liquid, is prevented. Preferably, the ammonium sulfate concentration in the feed of the oxidation reactor is lower than 40 wt.%, preferably lower than than 35 wt.%, and more preferably lower than 30 wt.%. Decreasing the ammonium sulfate concentration in the feed of the oxidation reactor has the advantage that more water can be allowed to evaporate, without the occurrence of crystallization in the purified aqueous liquid.

The gaseous oxidized products and purified aqueous liquid may be separated by any known means. Following separation of the gaseous oxidized products from the purified aqueous liquid, the purified aqueous liquid may be recycled to a crystallizer. The purified aqueous liquid may be directly recycled to the crystallizer, but recycling may also be done indirectly, for instance by combining the purified aqueous liquid with the mixture, in particular the neutralized Beckmann rearrangement mixture or by feeding the purified aqueous liquid to a step in which neutralization of a Beckmann rearrangement mixture is effected. The purified aqueous liquid and the gaseous oxidized products, may, with advantage, be used to heat the feed of the oxidation reactor or other steps in the production process of lactam before they are separated. Preferably, separation of the gaseous oxidized products and the purified aqueous liquid is performed, before the gaseous oxidized products and/or steam are used to heat the feed of the oxidation reactor or other steps in the production process of lactam.

Preferably, the process according to the invention involves obtaining ammonium sulfate crystals from the mixture by crystallization. This may be done by any means known to the skilled person. An ammonium sulfate solution may for instance be obtained by extracting the lactam from the mixture with an organic solvent. The organic solvent is preferably benzene or toluene. The extraction is preferably carried out at a temperature of between 20 and 60 °C. An ammonium sulfate solution may also be obtained by separating the ammonium sulfate solution phase and the aqueous lactam phase, preferably by phase separation. The ammonium sulfate solution thus obtained is preferably contacted with an organic solvent, such as for instance benzene or toluene, to recover small amounts of lactam which may have remained in the ammonium sulfate solution.

Ammonium sulfate crystals and mother liquor may be obtained from the ammonium sulfate solution in a crystallizer. Examples of suitable crystallizers are described in "Perry's Chemical Engineers Handbook", by Don W. Green and James O. Maloney, 7th edition, McGraw Hill, 1997, chapter 18, pages 44-55. Crystallizers which are operated by evaporation, optionally in combination with cooling, are particularly suitable for the process according to the invention. Usually, the crystallizer is operated at a temperature between 20 and 180 °C, and a pressure between 20 mbar and 8 bar. Preferably the crystallizer is operated at a temperature between 40 and 130 °C, and a pressure between 50 mbar and 2 bar. It is to be understood that it is also possible to use more than one crystallizer. In that case each of these crystallizers may be operated at a different pressure and/or temperature.

The ammonium sulfate concentration in the ammonium sulfate solution fed to the crystallizer is generally between 25 and 50 wt.%. The COD content of the ammonium sulfate solution fed to the crystallizer is generally between 0.1 and 20 g/kg in particular between 0.2 and 15 g/kg, and more in particular between 0.5 and 10 g/kg.

Mother liquor may be withdrawn from the crystallizer by any means known to the skilled person. A slurry comprising ammonium sulfate crystals and mother liquor may for instance be withdrawn from the crystallizer, and the mother liquor may be separated from the slurry, for instance by centrifuging. Preferably mother liquor which contains substantially no ammonium sulfate crystals is withdrawn from the crystallizer. The ammonium sulfate concentration of the mother liquor withdrawn from the crystallizer is usually between 35 and 60 wt.%, preferably between 38 and 55 wt.% and more preferably between 40 and 50 wt.%. The concentration organic impurities in the mother liquor withdrawn from the crystallizer usually corresponds to a COD content between 8 and 120 g/kg, preferably between 12 and 80 g/kg, and more preferably between 15 and 60 g/kg. Keeping the concentration of organic impurities in the mother liquor in the crystallizer low improves the quality of the ammonium sulfate crystals, i.e. they will have less color, they will contain fewer impurities, and the production of large crystals is facilitated. The lower limit for the COD content is not critical, and is mainly determined by process economics. The flow rate of the mother liquor withdrawn from the crystallizer is usually between 0.5 and 30 parts by volume per unit of time, preferably between 1 and 20 parts per unit of time for a flow rate of 100 parts by volume per unit of time for the ammonium sulfate solution which is fed to the crystallizer. If the flow rate of the mother liquor withdrawn from the crystallizer decreases, the COD content in the mother liquor generally increases, if the other circumstances are equal. If the feeding of the ammonium sulfate solution and/or the removal of mother liquor would occur discontinuously or batchwise, it is to be understood that the above mentioned flow rates are the average amounts fed or withdrawn per unit of time. If more than one crystallizer is used, it is advantageous to withdraw mother liquor from one crystallizer, feed said mother liquor to a subsequent crystallizer, in which the COD content may increase further, and withdraw the mother liquor with an increased COD content. This has the advantage that mother liquor with a relatively high COD content may be fed to the oxidation reactor, while it is avoided that the COD content of the mother liquor is at this high level in all crystallizers.

Preferably, lactam is recovered from the mixture, which preferably involves extracting the lactam with an organic solvent to form a lactam-containing organic liquid. This may be achieved by contacting the mixture or neutralized Beckmann rearrangement mixture with an organic solvent. It is also possible to obtain an aqueous lactam by separating the ammonium sulfate solution phase and the aqueous lactam phase. The aqueous lactam obtained may be contacted with an organic solvent, resulting in a lactam-containing organic liquid and an aqueous effluent. The organic solvent is preferably benzene or toluene. The lactam concentration in the lactam-containing organic liquid is preferably less than 30 wt.%. The extraction is preferably carried out at a temperature of between 20 and 60 °C.

The aqueous effluent usually contains between 0.1 and 10 wt.% ammonium sulfate dissolved in water, in particular between 0.2 and 8 wt.% ammonium sulfate dissolved in water, and more in particular between 0.5 and 6 wt.% ammonium sulfate dissolved in water. The COD content of the aqueous effluent is usually between 5 and 150 g/kg, in particular between 15 and 100 g/kg, and more in particular between 20 and 90 g/kg.

Lactam may be recovered from the lactam-containing organic liquid by any method known to the skilled person, for instance by distillation.

The process according to the invention is in particular suitable if the lactam contains 5 to 12 carbon atoms in the ring, and more in particular if the lactam is ε-caprolactam.

The invention is now illustrated with the aid of the following examples, without, however, being limited thereto.

### Brief description of the drawings

Figure 1 shows a flow chart of an embodiment (A) of the process according to the invention.
Figure 2 shows a flow chart of another embodiment (B) of the process according to the invention.
Figure 3 shows a flow chart of another embodiment (C) of the process according to the invention.
Figure 4 shows a flow chart of another embodiment (D) of the process according to the invention.

All COD contents mentioned in the examples are determined in accordance with the dichromate method according to ASTM D 1252-95.

### Example I

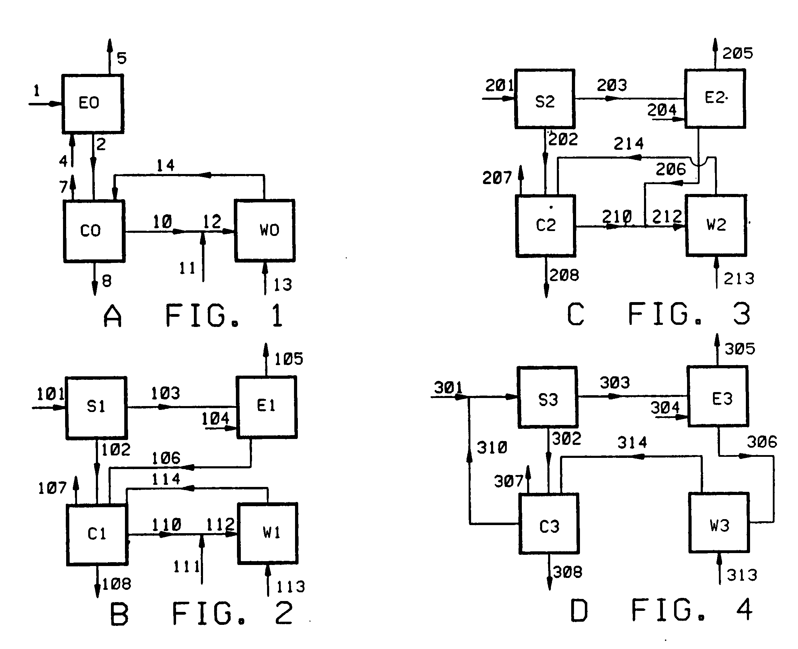

This example illustrates how the process may be carried out according to embodiment A. The example is described with reference to figure 1. The numbers in the text and figure refer to flows. Composition and flow rates of various flows are indicated in table 1.

Neutralized Beckmann rearrangement mixture **1** and benzene **4** are fed to extraction apparatus **E0** in which extraction caprolactam is effected. Caprolactam-containing organic liquid **5**, comprising caprolactam dissolved in benzene, and an ammonium sulfate solution **2** are withdrawn from extraction apparatus **E0.** Caprolactam is separated from the carpolactam-containing organic liquid using distillation. The ammonium sulfate solution **2** is fed to crystallizer **C0.** In crystallizer **C0** water is evaporated, and water vapour **7** and ammonium sulfate crystals **8** are withdrawn. Mother liquor **10** is withdrawn from crystallizer **C0,** and mixed with water **11** (aqueous dilution liquid), resulting in an oxidation mixture. Oxidation mixture **12** and air **13** are mixed, heated, pressurized, and fed to oxidation reactor **W0.** Oxidation reactor **W0** is operated at a pressure of 65 bar and at a temperature of 250 °C. In oxidation reactor **W0** oxidation of organic impurities is effected, resulting in gaseous oxidized products and purified ammonium sulfate solution. Upon separation of the purified ammonium solution and the gaseous oxidized products, a gaseous phase is obtained comprising steam and oxidized products (not shown). The gaseous phase is used to heat the feed of the oxidation reactor (not shown). Purified ammonium sulfate solution **14** is recycled to crystallizer **C0**.

In this example all streams which contain ammonium sulfate and organic impurities are recycled into the process. The COD content of the mother liquor in the crystallizer is kept at a low value, so that ammonium sulfate crystals with good quality are obtained. Crystallization in the purified ammonium sulfate solution does not occur.

### Example II

This example illustrates how the process may be carried out according to embodiment B. The example is described with reference to figure 2. The numbers in the text and in figure refer to flows. Composition and flow rates of various flows are indicated in table 2.

Neutralized Beckmann rearrangement mixture **101** is fed to separation apparatus **S1** in which separation of the ammonium sulfate solution phase and aqueous crude caprolactam phase is effected by phase separation. Ammonium sulfate solution **102** and aqueous crude caprolactam **103** are withdrawn from separation apparatus **S1.** Aqueous crude caprolactam **103** and benzene **104** are fed to extraction apparatus **E1,** in which extraction of caprolactam is effected. Caprolactam-containing organic liquid **105,** comprising caprolactam dissolved in benzene, and aqueous effluent **106** are withdrawn from extraction apparatus **E1.** Aqueous effluent **106** and ammonium sulfate solution **102** are mixed and fed to crystallizer **C1.** In crystallizer **C1** water is evaporated, and water vapour **107** and ammonium sulfate crystals **108** are withdrawn. Mother liquor **110** is withdrawn from crystallizer **C1,** and mixed with water **111** (aqueous dilution liquid), resulting in an oxidation mixture. Oxidation mixture **112** and air **113** are mixed, heated, pressurized, and fed to oxidation reactor **W1.** Oxidation reactor **W1** is operated at a pressure of 65 bar and at a temperature of 250 °C. In oxidation reactor **W1** oxidation of organic impurities is effected, resulting in gaseous oxidized products and purified ammonium sulfate solution. Upon separation of the purified ammonium solution and the gaseous oxidized products, a gaseous phase is obtained comprising steam and oxidized products (not shown). The gaseous phase is used to heat the feed of the oxidation reactor (not shown). Purified ammonium sulfate solution **114** is recycled to crystallizer **C1.**

In this example all streams which contain ammonium sulfate and organic impurities are recycled into the process. The COD content of the mother liquor in the crystallizer is kept at a low value, so that ammonium sulfate crystals with good quality are obtained. Crystallization in the purified ammonium sulfate solution does not occur.

### Example III

This example illustrates how the process may be carried out according to embodiment C. The example is described with reference to figure 3. The numbers in the text and figure refer to flows.

Neutralized Beckmann rearrangement mixture **201** is fed to separation apparatus **S2** in which separation of the ammonium sulfate solution phase and aqueous crude caprolactam phase is effected by phase separation. Aqueous crude caprolactam **203** and benzene **204** are fed to extraction apparatus **E2,** in which extraction of the caprolactam is effected. Caprolactam-containing organic liquid **205,** comprising caprolactam dissolved in benzene, and aqueous effluent **206** are withdrawn from extraction apparatus **E2.** Ammonium sulfate solution **202** is fed to crystallizer **C2.** In crystallizer **C2** water is evaporated, and water vapour **207** and ammonium sulfate crystals **208** are withdrawn. Mother liquor **210** is withdrawn from crystallizer **207** and mixed with aqueous effluent **206,** resulting in an oxidation mixture. Oxidation mixture **212** and air **213** are mixed, heated, pressurized, and fed to oxidation reactor **W2.** Oxidation reactor **W2** is operated at a pressure of 65 bar and at a temperature of 250 °C. In oxidation reactor **W2** oxidation of organic impurities is effected, resulting in gaseous oxidized products and purified ammonium sulfate solution. Upon separation of the purified ammonium sulfate solution and the oxidized products, a gaseous phase is obtained comprising steam and oxidized products (not shown). The gaseous phase is used to heat the feed of the oxidation reactor (not shown). Purified ammonium sulfate solution **204** is recycled to crystallizer **C2.**

In this example all streams which contain ammonium sulfate and organic impurities are recycled into the process. The COD content of the mother liquor in the crystallizer is kept at a low value, so that ammonium sulfate crystals with good quality are obtained. Crystallization in the purified ammonium sulfate solution does not occur. This example further shows that flow rates of mother liquor **210** and oxidation mixture **212** are considerably smaller than in examples I and II, so that a smaller oxidation reactor may be used. Since the mother liquor to be fed to the oxidation reactor is mixed with the aqueous effluent, no aqueous dilution liquid from external sources is needed to decrease the ammonium sulfate concentration of the feed of the oxidation reactor, and no water from external sources needs to be evaporated. Due to the COD content of the aqueous effluent **206,** the COD content of the oxidation mixture **212** is considerably higher than in examples I and II, which results in an energy-efficient operation of the wet air oxidation reactor.

### Example IV

This example illustrates how the process may be carried out according to embodiment D. The example is described with reference to figure 4. The numbers in the text and in figure 4 refer to flows. Composition and flow rates of various flows are indicated in table 4.

Neutralized Beckmann rearrangement mixture **301** and mother liquor **310** are combined and fed to separation apparatus **S3** in which separation of the ammonium sulfate solution phase and the aqueous crude caprolactam phase is effected by phase separation. Ammonium sulfate solution **302** and aqueous crude caprolactam **303** are withdrawn from separation apparatus **S3.** Aqueous crude caprolactam **303** and benzene **304** are fed to extraction apparatus **E3,** in which extraction of caprolactam is effected. Caprolactam-containing organic liquid **305,** comprising caprolactam dissolved in benzene, and aqueous effluent **306** are withdrawn from extraction apparatus **E3.** Caprolactam is separated from the caprolactam-containing organic liquid using distillation. Aqueous effluent **306** and air **313** are mixed, heated, pressurized, and fed to oxidation reactor **W3.** Oxidation reactor **W3** is operated at a pressure of 65 bar and at a temperature of 250 °C. In oxidation reactor **W3** oxidation of organic impurities is effected, resulting in gaseous oxidized products and purified effluent. Upon separation of the purified ammonium sulfate solution and the gaseous oxidized products, a gaseous phase is obtained comprising steam and oxidized products (not shown). The gaseous phase is used to heat the feed of the oxidation reactor (not shown). Purified effluent **314** and ammonium sulfate solution **302** are fed to crystallizer **C3.** In crystallizer **C3** water is evaporated, and water vapour **307** and ammonium sulfate crystals **308** are withdrawn. Mother liquor **310** is withdrawn from crystallizer **C3** and recycled to separation apparatus **S3.**

In this example all streams which contain ammonium sulfate and organic impurities are recycled into the process. The COD content of the mother liquor in the crystallizer is kept at a low value, so that ammonium sulfate crystals with good quality are obtained. Crystallization in the purified effluent does not occur. The flow rate of the aqueous effluent is small, so that a small oxidation reactor can be used. No aqueous dilution liquid from external sources is needed to decrease the ammonium sulfate concentration of the feed of the oxidation reactor, and no water from external sources needs to be evaporated. Due to the high COD content of the aqueous effluent the oxidation reactor is operated very efficiently.

**Table 1. Composition of flows in example I**

| Stream No. | Water (wt.%) | Amm. Sulf. (wt.%) | COD (g/kg) | Caprolactam (wt.%) | Flow rate (Weight parts/unit of time) |
|---|---|---|---|---|---|
| 2 | 61 | 39 | 7 | | 27 |
| 10 | 50 | 47.5 | 50 | | 4.7 |
| 11 | 100 | | | | 3.2 |
| 14 | 66 | 33.5 | 7 | | 6.7 |
| Oxid. Mixt. (12) | 70 | 28 | 30 | | 7.9 |

**Table 2. Composition of flows in example II**

| Stream No. | Water (wt.%) | Amm. Sulf. (wt.%) | COD (g/kg) | Caprolactam (wt.%) | Flow rate (Weight parts/unit of time) |
|---|---|---|---|---|---|
| 103 | 30 | 1 | | 69 | 7.5 |
| 102 | 58 | 42 | 3 | | 27 |
| 106 | 92.5 | 4 | 70 | | 2.2 |
| 110 | 50 | 47.5 | 50 | | 6 |
| 111 | 100 | | | | 4 |
| 114 | 66 | 33.5 | 7 | | 8.5 |
| Oxid. Mixt. (112) | 70 | 28 | 30 | | 10 |

**Table 3. Composition of flows in example III**

| Stream No. | Water (wt.%) | Amm. Sulf. (wt.%) | COD (g/kg) | Caprolactam (wt.%) | Flow rate (Weight parts/unit of time) |
|---|---|---|---|---|---|
| 203 | 30 | 1 | | 69 | 7.5 |
| 202 | 58 | 42 | 3 | | 27 |
| 210 | 50 | 47.5 | 50 | | 2.8 |
| 206 | 92.5 | 4 | 70 | | 2.2 |
| 214 | 58 | 42 | 12 | | 3.5 |
| Oxid. Mixt. (212) | 69 | 28 | 59 | | 5.0 |

**Table 4. Composition of flows in example IV**

| Stream No. | Water (wt.%) | Amm. Sulf. (wt.%) | COD (g/kg) | Caprolactam (wt.%) | Flow rate (Weight parts/unit of time) |
|---|---|---|---|---|---|
| 303 | 30 | 1 | | 69 | 7.5 |
| 302 | 58 | 42 | 3 | | 27 |
| 310 | 50 | 47.5 | 50 | | 3.5 |
| 314 | 92 | 6.5 | 20 | | 1.4 |
| Aq. efl. (306) | 92 | 3 | 100 | | 3.0 |

## Claims

1. Process for treating a mixture comprising:
i) an ammonium sulfate solution phase containing first organic impurities; and
ii) an aqueous lactam phase containing second organic impurities;
wherein said process comprises:
forming an aqueous liquid containing the first and second organic impurities, and
subjecting the aqueous liquid to a wet air oxidation process to purify the aqueous liquid.

2. Process according to claim 1, wherein the mixture is a neutralized Beckmann rearrangement mixture.

3. Process according to claim 1 or claim 2, wherein the process further comprises:
obtaining ammonium sulfate crystals from the mixture by crystallization.

4. Process according to any one of claim 1 to 3, wherein the process further comprises:
extracting the lactam with an organic solvent to form a lactam-containing organic liquid.

5. Process according to any one of claims 1 to 4, wherein the aqueous liquid contains ammonium sulfate.

6. Process according to claim 5, wherein the process further comprises:
recycling the purified aqueous liquid to a crystallizer.

7. Process according to any one of claims 1 to 6, wherein the process comprises the step of:
Aa) contacting the mixture with an organic solvent, resulting in a lactam-containing organic liquid and an ammonium sulfate solution.

8. Process according to claim 7, wherein the process comprises the steps of:
Ab) processing the ammonium sulfate solution through a crystallizer to form ammonium sulfate crystals and a mother liquor
Ac) combining the mother liquor with aqueous dilution liquid, resulting in an oxidation mixture
Ad) subjecting the oxidation mixture to a wet air oxidation process, resulting in purified ammonium sulfate solution.

9. Process according to any one of claims 1 to 6, wherein the process comprises the steps of:
Ba) separating the ammonium sulfate solution phase and the aqueous lactam phase, resulting in an ammonium sulfate solution and aqueous lactam
Bb) contacting the aqueous lactam with an organic solvent, resulting in a lactam-containing organic liquid and an aqueous effluent
Bc) combining the ammonium sulfate solution the aqueous effluent.

10. Process according to claim 9, wherein the process comprises the steps of:
Bd) processing the combined ammonium sulfate solution and aqueous effluent through a crystallizer to form ammonium sulfate crystals and a mother liquor
Be) combining the mother liquor with aqueous dilution liquid, resulting in an oxidation mixture
Bf) subjecting the oxidation mixture to a wet air oxidation process, resulting in purified ammonium sulfate solution.

11. Process according to any one of claims 1 to 6, wherein the process comprises the steps of:
Ca) separating the ammonium sulfate solution phase and the aqueous lactam phase, resulting in an ammonium sulfate solution and an aqueous lactam
Cb) contacting the aqueous lactam with an organic solvent, resulting in a lactam-containing organic liquid and an aqueous effluent
Cc) processing the ammonium sulfate solution through a crystallizer to obtain ammonium sulfate crystals and a mother liquor
Cd) combining the mother liquor and the aqueous effluent, resulting in an oxidation mixture
Ce) subjecting the oxidation mixture to a wet air oxidation process, resulting in purified ammonium sulfate solution.

12. Process according to any one of claims 1 to 6, wherein the process comprises the steps of:
Da) separating the ammonium sulfate solution phase and the aqueous lactam phase, resulting in an ammonium sulfate solution and an aqueous lactam
Db) contacting the aqueous lactam with an organic solvent, resulting in a lactam-containing organic liquid and an aqueous effluent
Dc) processing the ammonium sulfate solution through a crystallizer to obtain ammonium sulfate crystals and mother liquor
Dd) combining the mother liquor with the mixture
De) subjecting the aqueous effluent to a wet air oxidation process, resulting in purified effluent.

13. Process according to any one of claims 1 to 12,
wherein said lactam is caprolactam.

14. Process for the production of ammonium sulfate crystals from a mixture, in particular a neutralized Beckmann rearrangement mixture, comprising
i) an ammonium sulfate solution phase containing first organic impurities; and
ii) an aqueous lactam phase containing second organic impurities;
which process involves obtaining ammonium sulfate crystals from the mixture by crystallization, and wherein the process is carried out according to any one of claims 1 to 13.

## Patentansprüche

1. Verfahren zur Behandlung eines Gemischs, enthaltend:
i) eine Ammoniumsulfatlösungsphase, die erste organische Verunreinigungen enthält; und
ii) eine wäßrige Lactamphase, die zweite organische Verunreinigungen enthält;
bei dem man:
eine wäßrige Flüssigkeit, die die ersten und zweiten organischen Verunreinigungen enthält, bildet und
die wäßrige Flüssigkeit einem Naßluftoxidationsverfahren zur Reinigung der wäßrigen Flüssigkeit unterwirft.

2. Verfahren nach Anspruch 1, bei dem es sich bei dem Gemisch um ein neutralisiertes Gemisch aus der Beckmann-Umlagerung handelt.

3. Verfahren nach Anspruch 1 oder Anspruch 2, bei dem man ferner:
durch Kristallisation aus dem Gemisch Ammoniumsulfatkristalle erhält.

4. Verfahren nach einem der Ansprüche 1 bis 3, bei dem man ferner:
das Lactam mit einem organischen Lösungsmittel extrahiert, wobei man eine lactamhaltige organische Flüssigkeit erhält.

5. Verfahren nach einem der Ansprüche 1 bis 4, bei dem die wäßrige Flüssigkeit Ammoniumsulfat enthält.

6. Verfahren nach Anspruch 5, bei dem man ferner:
die gereinigte wäßrige Flüssigkeit in einen Kristallisator zurückführt.

7. Verfahren nach einem der Ansprüche 1 bis 6, bei dem man:
Aa) das Gemisch mit einem organischen Lösungsmittel in Berührung bringt, wobei man eine lactamhaltige organische Flüssigkeit und eine Ammoniumsulfatlösung erhält.

8. Verfahren nach Anspruch 7, bei dem man:
Ab) die Ammoniumsulfatlösung über einen Kristallisator verarbeitet, wobei man Ammoniumsulfatkristalle und eine Mutterlauge erhält,
Ac) die Mutterlauge mit wäßriger Verdünnungsflüssigkeit vereinigt, wobei man ein Oxidationsgemisch erhält,
Ad) das Oxidationsgemisch einem Naßluftoxidationsverfahren unterwirft, wobei man eine gereinigte Ammoniumsulfatlösung erhält.

9. Verfahren nach einem der Ansprüche 1 bis 6, bei dem man:
Ba) die Ammoniumsulfatlösungsphase und die wäßrige Lactamphase trennt, wobei man eine Ammoniumsulfatlösung und wäßriges Lactam erhält,
Bb) das wäßrige Lactam mit einem organischen Lösungsmittel in Berührung bringt, wobei man eine lactamhaltige organische Flüssigkeit und einen wäßrigen Austragsstrom erhält,
Bc) die Ammoniumsulfatlösung mit dem wäßrigen Austragsstrom vereinigt.

10. Verfahren nach Anspruch 9, bei dem man:
Bd) die Ammoniumsulfatlösung und den wäßrigen Austragsstrom nach Vereinigung über einen Kristallisator verarbeitet, wobei man Ammoniumsulfatkristalle und eine Mutterlauge erhält,
Be) die Mutterlauge mit wäßriger Verdünnungsflüssigkeit vereinigt, wobei man ein Oxidationsgemisch erhält,
Bf) das Oxidationsgemisch einem Naßluftoxidationsverfahren unterwirft, wobei man eine gereinigte Ammoniumsulfatlösung erhält.

11. Verfahren nach einem der Ansprüche 1 bis 6, bei dem man:
Ca) die Ammoniumsulfatlösungsphase und die wäßrige Lactamphase trennt, wobei man eine Ammoniumsulfatlösung und wäßriges Lactam erhält, Cb) das wäßrige Lactam mit einem organischen Lösungsmittel in Berührung bringt, wobei man eine lactamhaltige organische Flüssigkeit und einen wäßrigen Austragsstrom erhält,
Cc) die Ammoniumsulfatlösung über einen Kristallisator verarbeitet, wobei man Ammoniumsulfatkristalle und eine Mutterlauge erhält,
Cd) die Mutterlauge und den wäßrigen Austragsstrom vereinigt, wobei man ein Oxidationsgemisch erhält,
Ce) das Oxidationsgemisch einem Naßluftoxidationsverfahren unterwirft, wobei man eine gereinigte Ammoniumsulfatlösung erhält.

12. Verfahren nach einem der Ansprüche 1 bis 6, bei dem man:
Da) die Ammoniumsulfatlösungsphase und die wäßrige Lactamphase trennt, wobei man eine Ammoniumsulfatlösung und wäßriges Lactam erhält, Db) das wäßrige Lactam mit einem organischen Lösungsmittel in Berührung bringt, wobei man eine lactamhaltige organische Flüssigkeit und einen wäßrigen Austragsstrom erhält,
Dc) die Ammoniumsulfatlösung über einen Kristallisator verarbeitet, wobei man Ammoniumsulfatkristalle und eine Mutterlauge erhält,
Dd) die Mutterlauge mit dem Gemisch vereinigt,
Ce) den wäßrigen Austragsstrom einem Naßluftoxidationsverfahren unterwirft, wobei man einen gereinigten Austragsstrom erhält.

13. Verfahren nach einem der Ansprüche 1 bis 12, bei dem es sich bei dem Lactam um Caprolactam handelt.

14. Verfahren zur Herstellung von Ammoniumsulfatkristallen aus einem Gemisch, insbesondere einem neutralisierten Gemisch aus der Beckmann-Umlagerung, enthaltend:
i) eine Ammoniumsulfatlösungsphase, die erste organische Verunreinigungen enthält; und
ii) eine wäßrige Lactamphase, die zweite organische Verunreinigungen enthält;
bei dem man durch Kristallisation aus dem Gemisch Ammoniumsulfatkristalle erhält und das gemäß einem der Ansprüche 1 bis 13 durchgeführt wird.

## Revendications

1. Procédé de traitement d'un mélange comprenant :
i) une phase en solution de sulfate d'ammonium contenant des premières impuretés organiques ; et
ii) une phase lactame aqueuse contenant des deuxièmes impuretés organiques ;
ledit procédé comprenant les étapes consistant à :
former un liquide aqueux contenant les premières et deuxièmes impuretés organiques, et
soumettre le liquide aqueux à un procédé d'oxydation à l'air humide en vue de purifier le liquide aqueux.

2. Procédé selon la revendication 1, dans lequel le mélange est un mélange de réarrangement de Beckmann neutralisé.

3. Procédé selon la revendication 1 ou la revendication 2, le procédé comprenant en outre l'étape consistant à :
obtenir des cristaux de sulfate d'ammonium à partir du mélange par cristallisation.

4. Procédé selon l'une quelconque des revendications 1 à 3, le procédé comprenant en outre l'étape consistant à :
extraire le lactame avec un solvant organique en vue de former un liquide organique contenant du lactame.

5. Procédé selon l'une quelconque des revendications 1 à 4, dans lequel le liquide aqueux contient du sulfate d'ammonium.

6. Procédé selon la revendication 5, le procédé comprenant en outre l'étape consistant à :
recycler le liquide aqueux purifié vers un cristalliseur.

7. Procédé selon l'une quelconque des revendications 1 à 6, le procédé comprenant l'étape consistant à :
Aa) mettre en contact le mélange avec un solvant organique, en donnant lieu à un liquide organique contenant du lactame et à une solution de sulfate d'ammonium.

8. Procédé selon la revendication 7, le procédé comprenant les étapes consistant à :
Ab) traiter la solution de sulfate d'ammonium à travers un cristalliseur en vue de former des cristaux de sulfate d'ammonium et une liqueur mère,
Ac) combiner la liqueur mère avec un liquide de dilution aqueux, en donnant lieu à un mélange d'oxydation,
Ad) soumettre le mélange d'oxydation à un procédé d'oxydation à l'air humide, en donnant lieu à une solution de sulfate d'ammonium purifiée.

9. Procédé selon l'une quelconque des revendications 1 à 6, le procédé comprenant les étapes consistant à :
Ba) séparer la phase en solution de sulfate d'ammonium et la phase lactame aqueuse, en donnant lieu à une solution de sulfate d'ammonium et à un lactame aqueux,
Bb) mettre en contact le lactame aqueux avec un solvant organique, en donnant lieu à un liquide organique contenant du lactame et à un effluent aqueux,
Bc) combiner la solution de sulfate d'ammonium et l'effluent aqueux.

10. Procédé selon la revendication 9, le procédé comprenant les étapes consistant à :
Bd) traiter la solution de sulfate d'ammonium et l'effluent aqueux combinés à travers un cristalliseur en vue de former des cristaux de sulfate d'ammonium et une liqueur mère,
Be) combiner la liqueur mère avec un liquide de dilution aqueux, en donnant lieu à un mélange d'oxydation,
Bf) soumettre le mélange d'oxydation à un procédé d'oxydation à l'air humide, en donnant lieu à une solution de sulfate d'ammonium purifiée.

11. Procédé selon l'une quelconque des revendications 1 à 6, le procédé comprenant les étapes consistant à :
Ca) séparer la phase en solution de sulfate d'ammonium et la phase lactame aqueuse, en donnant lieu à une solution de sulfate d'ammonium et à un lactame aqueux,
Cb) mettre en contact le lactame aqueux avec un solvant organique, en donnant lieu à un liquide organique contenant du lactame et à un effluent aqueux,
Cc) traiter la solution de sulfate d'ammonium à travers un cristalliseur en vue d'obtenir des cristaux de sulfate d'ammonium et une liqueur mère,
Cd) combiner la liqueur mère et l'effluent aqueux, en donnant lieu à un mélange d'oxydation,
Ce) soumettre le mélange d'oxydation à un procédé d'oxydation à l'air humide, en donnant lieu à une solution de sulfate d'ammonium purifiée.

12. Procédé selon l'une quelconque des revendications 1 à 6, le procédé comprenant les étapes consistant à :
Da) séparer la phase en solution de sulfate d'ammonium et la phase lactame aqueuse, en donnant lieu à une solution de sulfate d'ammonium et à un lactame aqueux,
Db) mettre en contact le lactame aqueux avec un solvant organique, en donnant lieu à un liquide organique contenant du lactame et à un effluent aqueux,
Dc) traiter la solution de sulfate d'ammonium à travers un cristalliseur en vue d'obtenir des cristaux de sulfate d'ammonium et une liqueur mère,
Dd) combiner la liqueur mère avec le mélange,
De) soumettre l'effluent aqueux à un procédé d'oxydation à l'air humide, en donnant lieu à un effluent purifié.

13. Procédé selon l'une quelconque des revendications 1 à 12, dans lequel ledit lactame est le caprolactame.

14. Procédé de production de cristaux de sulfate d'ammonium à partir d'un mélange, en particulier d'un mélange de réarrangement de Beckmann neutralisé, comprenant
i) une phase en solution de sulfate d'ammonium contenant des premières impuretés organiques ; et
ii) une phase lactame aqueuse contenant des deuxièmes impuretés organiques ;
lequel procédé met en jeu l'obtention de cristaux de sulfate d'ammonium à partir du mélange par cristallisation, et le procédé étant réalisé conformément à l'une quelconque des revendications 1 à 13.
